# EUROPÄISCHE PATENTANMELDUNG

(11) **EP 1 632 193 A1**
(43) Veröffentlichungstag der Anmeldung: **08.03.2006**
(21) Anmeldenummer: 04020839.9
(22) Anmeldetag: 02.09.2004
(51) Int. Cl.: A61B 19/00, A61B 5/107

(54) **Hüftregistrierungssystem**

(71) Anmelder: BrainLAB AG, 85551 Kirchheim/Heimstetten (DE)
(72) Erfinder: Drumm, Peter, 81543 München (DE); Brecht, Gunther, 85609 Dornach (DE)
(74) Vertreter: Schwabe - Sandmair - Marx

(57) **Zusammenfassung**

Die Erfindung bezieht sich auf eine Vorrichtung zum Registrieren einer Hüfte eines Patienten mit Markern 5 und Positionierungselementen 4, welche auf charakteristische Punkte der Hüfte aufgesetzt werden können, wobei nach dem Aufsetzen der Positionierungselemente 4 auf charakteristische Punkte der Hüfte aus der Position der Marker 5 Daten zur Registrierung der Hüfte ermittelt werden können, sowie auf ein Verfahren zum Registrieren einer Hüfte mit einer solchen Vorrichtung, wobei die Position der auf der Vorrichtung 1 angeordneten Marker 5 erfasst und daraus Daten zur Registrierung der Hüfte ermittelt werden.

## Beschreibung

Die vorliegende Erfindung bezieht sich auf eine Vorrichtung, ein System und ein Verfahren zum Registrieren einer Hüfte. Insbesondere bezieht sich die Erfindung auf das Registrieren, das heißt das Bestimmen der räumlichen Lage, der Hüfte bzw. der Beckenknochen zum Beispiel zur Vorbereitung eines chirurgischen Eingriffs.

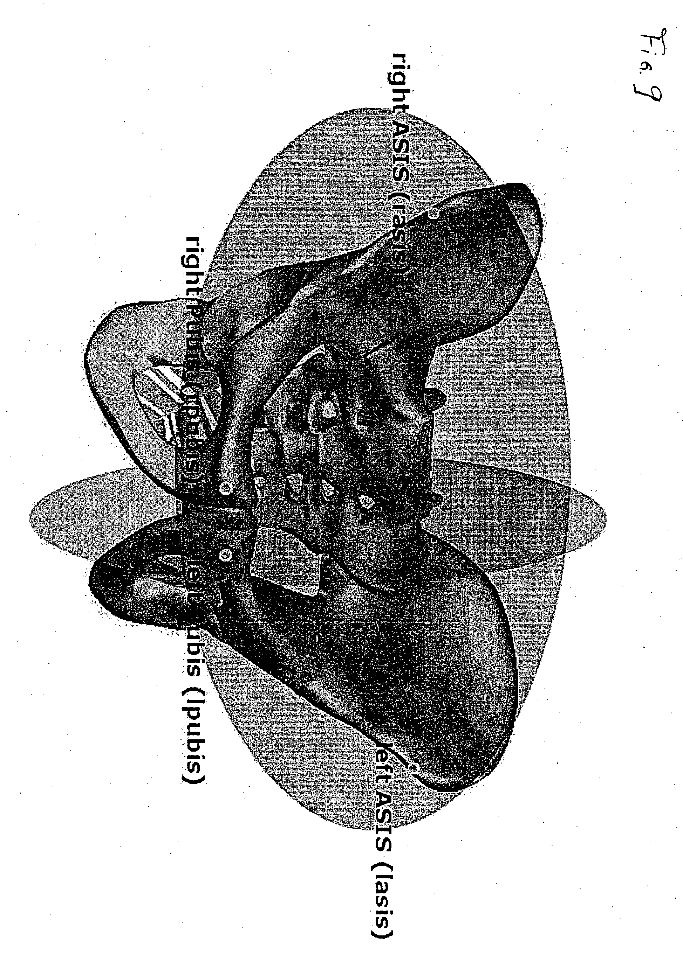

Zur Vorbereitung eines chirurgischen Eingriffs im Bereich der Hüfte und insbesondere bei einer Operation an einem Beckenknochen zum Beispiel im Bereich des Hüftgelenks, wie zum Beispiel beim Einsetzen einer Hüftgelenkpfanne, ist es erforderlich, dass die Hüfte registriert wird, das heißt, dass die Position oder Lage der Hüfte bzw. der Beckenknochen im Raum erfasst wird. Bei der Registrierung einer Hüfte können die räumliche Lage der Midsagittalebene, die eine Symmetrieebene des Beckens ist, und der Beckeneingangsebene bzw. anterioren Beckenebene, welche durch zwei Spinapunkte und einen Pubispunkt definiert ist, ermittelt werden. Ein Spinapunkt, häufig auch als ASIS (Anterior Superior Illiac Spine) bezeichnet, ist ein charakteristischer Punkt bzw. eine anatomische Landmarke auf einem Beckenknochen und kann auf dem Beckenknochen leicht aufgefunden werden. In Figur 9 sind der linke Spinapunkt (lasis) und der rechte Spinapunkt (rasis) eingezeichnet. Ein Pubispunkt ist ein charakteristischer Punkt auf dem Schamberg eines Beckenknochens und ist in Figur 9 mit rpubis für den Pubispunkt des rechten Beckenknochens und mit lpubis für den Pubispunkt des linken Beckenknochens bezeichnet.

Zum Registrieren der Hüfte wird der Patient in Rückenlage gebracht, die Positionen der Spina- und Pubispunkte werden von einem Chirurgen ertastet und zum Beispiel mittels eines mit Markern versehenen Pointers, welche von einer Kamera erfasst werden können, einer Navigationssoftware mitgeteilt. Nachdem die entsprechenden Punkte in Rückenlage des Patienten aufgenommen wurden, wird der Patient in Seitenlage gebracht, um zum Beispiel im Bereich des rechten oder linken Beckenknochens einen chirurgischen Eingriff vorzunehmen, wie zum Beispiel eine künstliche Hüftgelenkpfanne einzusetzen, deren Position durch die mittels der Spina- und Pubispunkte definierte Lage der Midsagittalebene und der anterioren Beckenebene festgelegt wird.

Treten bei der Bestimmung der Spina- und Pubispunkte Ungenauigkeiten auf oder ergibt sich eine Veränderung der Lage der Beckenknochen beim Drehen des Patienten von der Rückenlage in die Seitenlage, so kann es zu einer Ungenauigkeit beim Einsetzen eines künstlichen Hüftgelenks kommen, was zur Folge haben kann, dass Komplikationen beim Patienten auftreten, da das künstliche Hüftgelenk beispielsweise nicht richtig positioniert eingesetzt wurde und Schmerzen verursacht. Ebenso kann aufgrund einer nicht genau positionierten Hüftgelenkpfanne ein hoher Verschleiß verursacht werden, so dass die Lebensdauer eines Implantats verringert wird.

Es ist eine Aufgabe der vorliegenden Erfindung eine Vorrichtung, ein System und ein Verfahren zum Registrieren einer Hüfte vorzuschlagen, welche eine präzisere Registrierung der Hüfte ermöglichen.

Diese Aufgabe wird durch die Vorrichtung, das System und das Verfahren gemäß den unabhängigen Patentansprüchen gelöst. Vorteilhafte Ausführungsformen ergeben sich aus den Unteransprüchen.

Die erfindungsgemäße Vorrichtung zum Registrieren einer Hüfte ermöglicht das Registrieren der Hüfte in einer Patientenseitenlage, das heißt ein Patient muss nach dem Registrieren der Hüfte nicht mehr von der Rücken- in die Seitenlage gedreht werden und weist erfindungsgemäß Marker, wie zum Beispiel vier passive Marker auf, die beispielsweise durch reflektierende Oberflächen oder Kugeln gebildet werden können, und weist weiterhin Positionierungselemente, wie zum Beispiel drei oder vier Stäbe mit einem konisch oder spitz zulaufenden Ende auf, welche zum Registrieren der Hüfte mit der erfindungsgemäßen Vorrichtung auf charakteristische Punkte der Hüfte, wie zum Beispiel Spinapunkte (ASIS) und Pubispunkte aufgesetzt werden können, wobei die auf der Vorrichtung angebrachten Marker in einem definierten und bekannten Lageverhältnis zu den Positionierungselementen bzw. Spitzen oder Enden der Stäbe stehen. Dabei müssen die Positionierungselemente nicht unmittelbar mit einem Beckenknochen in Kontakt kommen und können auch auf die Haut eines Patienten aufgesetzt werden, durch welche hindurch die charakteristischen Punkte ertastet werden können. Bei einer mit den Positionierungselementen auf charakteristische Punkte einer Hüfte bzw. Beckenknochen aufgesetzten erfindungsgemäßen Vorrichtung kann durch die Lage der mit der Vorrichtung verbundenen Vorrichtung Marker bestimmt werden, wie zum Beispiel die Midsagittalebene und die Beckeneingangsebene verläuft bzw. wie deren räumliche Lage ist, wodurch die Hüfte registriert ist. Vorteilhaft sind an der Hüfte bzw. einem Beckenknochen ebenfalls Marker befestigt.

Mit der erfindungsgemäßen Vorrichtung kann somit durch die eindeutig bestimmbare Zuordnung der räumlichen Position von Aufsetzpunkten der Positionierungselemente zu den Positionen der mit der Vorrichtung verbundenen Marker zum Beispiel die Position eines oder beider Spinapunkte und eines oder beider Pubispunkte der Beckenknochen und basierend darauf die räumliche Lage der Midsagittalebene als Symmetrieebene und die räumliche Lage der Beckeneingangsebene, welche zum Beispiel durch die Lage zweier Spinapunkte und eines Pubispunktes definiert ist, bestimmt werden. Somit kann eine Hüfte in Patientenseitenlage ohne die Durchführung von Röntgenaufnahmen registriert werden, wobei vorteilhaft der Patient in Seitenlage bereits durch eine Abstützvorrichtung, wie zum Beispiel durch zwei mit einem Polster besetzte Platten oder ein Gestell, stabil und bevorzugt im Wesentlichen im Bereich der Hüfte unbeweglich gelagert ist. Zum Registrieren der Hüfte bzw. der Beckenknochen eines Patienten kann die erfindungsgemäße Vorrichtung zum Beispiel an einer Abstützvorrichtung zur Positionierung des Patienten in Seitenlage befestigt werden, wobei die Vorrichtung dann am Patienten so positioniert und mit der Vorrichtung verbundene Positionierungselemente so verschoben werden, dass diese auf charakteristischen Punkten der Beckenknochen aufliegen oder aufsetzen, um anhand der mit der Vorrichtung verbundenen Marker die Lage der Midsagittalebene und der Beckeneingangsebene zu bestimmen. Nach dem Registrieren der Hüfte kann die erfindungsgemäße Vorrichtung wieder von der Abstützvorrichtung abgenommen werden, so dass ein chirurgischer Eingriff zum Beispiel zum Einsetzen einer Gelenkpfanne vorgenommen werden kann. Die Ausrichtung der Gelenkpfanne wird durch die Lage der mit der Vorrichtung bestimmbaren Midsagittalebene und der Beckeneingangsebene festgelegt, wobei ein Winkel, welchen eine Normale der Gelenkpfanne zur Midsagittalebene bildet, als Inklination und welchen die Normale der Gelenkpfanne zur Beckeneingangsebene bildet, als Anteversion bezeichnet wird. Die Gelenkpfanne wird so positioniert, dass diese Winkel, welche von Patient zu Patient varriieren, einen gewünschten Wert von zum Beispiel 45° (Inklination) und zum Beispiel 15° (Anteversion) haben.

Die Erfassung der Position der auf der Vorrichtung angeordneten Marker kann durch bekannte Navigationssysteme, wie zum Beispiel Vector Vision (R), zum Beispiel mittels Infrarotkameras erfolgen und wird im Detail nicht näher beschrieben.

Die erfindungsgemäße Vorrichtung kann ein Gestell, ein Rahmen oder einfach eine Platte sein, auf welcher zum Beispiel vier Positionierungselemente, wie zum Beispiel vier Stäbe, fest oder verschiebbar angeordnet sind. Beispielsweise kann mit einem Verstellelement der Abstand zwischen zwei Positionierungselementen, welche auf die Spinapunkte aufgesetzt werden sollen, verändert werden, wobei beispielsweise ein Positionierungselement fest mit der Vorrichtung verbunden ist und das andere Positionierungselement relativ zur Vorrichtung bewegbar ist. Ebenso ist es möglich, dass beide Positionierungselemente relativ zur Vorrichtung bewegbar sind, also beispielsweise entlang von Führungen in der Vorrichtung verschoben werden können, wobei zum Beispiel ein definierter Punkt auf der Vorrichtung vorgegeben ist, an welchem auch ein Marker angeordnet sein kann, welcher bevorzugt immer in der Mitte zwischen den beiden Positionierungselementen liegt. Durch eine Mechanik kann zum Beispiel sichergestellt werden, dass bei der Verschiebung eines Positionierungselementes das andere Element in die entgegengesetzte Richtung bewegt wird. Entsprechend können auch die Positionierungselemente, welche auf den Pubispunkten aufgesetzt werden sollen, fest oder verschiebbar mit der Vorrichtung verbunden sein und ebenfalls zum Beispiel einen Mechanismus aufweisen, welcher es ermöglicht, dass die Positionierungselemente immer symmetrisch bezüglich eines definierten Mittelpunktes, an welchem ein Marker angebracht werden kann, bewegt werden. Eine Mechanik, welche eine synchrone Bewegung der Positionierungselemente bezüglich eines Mittelpunktes ermöglicht, ist zur Registrierung einer Hüfte vorteilhaft, da zum Beispiel an diesem Mittelpunkten Marker angebracht werden können und die Midsagittalebene durch die einfach bestimmbaren Positionen dieser Marker verläuft.

Allgemein können die Marker an jeder beliebigen von einer Kamera erfassbaren Position der Vorrichtung und/oder der Positionierungselmente angebracht werden und sollten vorteilhaft ein definiertes oder definierbares Lageverhältnis zur Vorrichtung oder zu den mit der Vorrichtung verbundenen Positionierungselementen aufweisen, um zum Beispiel mittels einer Datenbank, in welcher die Geometrie der Vorrichtung und Verstellmöglichkeiten der Positionierungselemente abgespeichert sind, ermitteln zu können, wo die Position der Aufsetzpunkte der Positionierungselemente ist, wenn die räumliche Position der Marker ermittelt wurde.

Beispielsweise kann mit der Vorrichtung ein bekannter Referenzstern, an welchem drei Marker angebracht sind, verbunden werden, wobei vorteilhaft die Sternebene, also die Ebene, in welcher die mit dem Stern verbundenen Marker liegen, parallel zu einer Ebene ist, welche durch die vier Aufsetzpunkte der vier Positionierungselemente definiert wird. Dabei können die Positionierungselemente durch eine Führung in der Vorrichtung bevorzugt nur so bewegt werden, dass sich deren Aufsetzpunkte immer innerhalb einer Ebene bewegen, welche im auf den Beckenknochen aufgesetzten Zustand der Vorrichtung die Beckeneingangsebene (anteriore Beckenebene) definiert, so dass, wenn der Abstand zwischen der Sternebene und der durch die Aufsetzpunkte der Positionierungselemente definierten Ebene bekannt ist, die erfindungsgemäße Vorrichtung also präkalibriert ist, aus den erfassten Positionen der mit dem Stern verbundenen Marker einfach die Lage der Beckeneingangsebene ermittelt werden kann.

Vorzugsweise kann der Abstand eines Positionierungselementpaares zum Anlegen an die Spinapunkte von dem Positionierungselement zum Anlegen an die Pubispunkte verändert werden. Vorteilhaft kann ein Positionierungselementpaar so verschoben werden, dass eine durch die Anlagepunkte eines Positionierungselementpaares definierte Linie immer parallel ist zu einer durch die Anlagepunkte des anderen Positionierungselementpaares definierte Linie. Beispielsweise kann ein Positionierungselementpaar in der Vorrichtung so geführt werden, dass der Abstand zwischen den Positionierungselementen verändert werden kann, wohingegen ein zweites Positionierurigselementpaar auf einem Schlitten angeordnet ist, welcher relativ zur Vorrichtung verschoben werden kann und wobei der Abstand der auf dem Schlitten angeordneten und in dem Schlitten geführten Positionierungselemente ebenfalls verändert werden kann.

Es ist auch möglich, dass einzelne Marker auf der Vorrichtung selbst und/oder an einem oder mehreren verschiebbaren Verstellelementen angebracht werden. Beispielsweise kann jeweils ein Marker an einem Positionierungselement angebracht werden, so dass bei vier verwendeten Positionierungselementen insgesamt vier Marker vorhanden sind. Ebenso ist es möglich, dass zum Beispiel auf nur zwei Positionierungselementen Marker angebracht sind und jeweils ein weiterer Marker zwischen zwei Positionierungselementen mittig angeordnet ist, zum Beispiel auf einem Einstellelement, beispielsweise einen Drehrad, mit welchem der Abstand zweier Marker voneinander eingestellt werden kann. Vorteilhaft sind die Positionierungselemente so verschiebbar in der Vorrichtung gelagert, dass der durch die zwei fest mit der Vorrichtung verbundenen Marker und einen weiteren Marker gebildete Winkel immer 90 Grad beträgt.

Ein Marker und/oder Referenzstern kann mit der Vorrichtung auch mittels einer verstellbaren Markeranordnung verbunden werden, welche in der Europäischen Patentanmeldung mit der Anmeldenr. 04 003 019.9 und der korrespondierenden US Provisional Application Nr. 60/548,153 beschrieben ist, deren Lehren bezüglich der Ausbildung einer verstellbaren Markeranordnung in diese Anmeldung aufgenommen werden. Eine in den genannten Schriften verstellbare Markeranordnung ermöglicht es, dass ein oder mehrere Marker oder ein Referenzstern mit der Vorrichtung oder einem oder mehreren Positionierungselementen verbunden werden und unabhängig von der Bewegung der Vorrichtung oder des Positionierungselements bewegt werden können, ohne dass die räumliche Zuordnung eines oder mehrerer Marker zu den mit dem oder den Markern verbundenen Elementen verloren geht.

Gemäß einem weiteren Aspekt bezieht sich die Erfindung auf ein System zum Registrieren einer Hüfte eines Patienten mit einer wie oben beschriebenen Vorrichtung, einem Erfassungsgerät, wie zum Beispiel einer Infrarot-Kamera, zum Erfassen der Position der mit der Vorrichtung verbundenen Marker und einer mit der Erfassungsvorrichtung verbundenen Recheneinheit, welche aus der erfassten Position der Marker die Position der Anlagepunkte der Positionierungselemente und damit die Position der Spinapunkte und Pubispunkte ermitteln und so die Lage der Midsagittalebene und der Beckeneingangsebene bestimmen kann. Vorteilhaft ist die Recheneinheit mit einer Datenbank verbunden, in welcher die Geometrie einer oder mehrerer erfindungsgemäßer Vorrichtungen abgespeichert ist, um beispielsweise verschiedene Vorrichtungen verwenden zu können, wobei aus der Position der Marker die Position der Positionierungselemente berechnet werden kann.

Vorteilhaft ist die Recheneinheit mit einer Ausgabeeinheit, wie zum Beispiel einem Bildschirm, verbunden, an welchem zum Beispiel die mit dem erfindungsgemäßen System ermittelte Lage der Midsagittalebene und der Beckeneingangsebene angezeigt werden kann, wie beispielsweise in Figur 9 dargestellt.

Gemäß einem weiteren Aspekt bezieht sich die Erfindung auf ein Verfahren zum Registrieren einer Hüfte mit einer wie oben beschriebenen Vorrichtung, wobei Aufsetzpunkte der Positionierungselemente auf charakteristischen Punkten der Beckenknochen aufgesetzt werden, die Positionen der mit der Vorrichtung verbundenen Marker erfasst werden und aus den erfassten Positionen der Marker die Positionen der Aufsetzpunkte der Positionierungselemente bestimmt werden.

Des weiteren bezieht sich die Erfindung auf ein Computerprogramm, welches, wenn es in einem Computer geladen ist oder auf einem Computer läuft, ein solches Verfahren ausführt. Weiterhin bezieht sich die Erfindung auf ein Programmspeichermedium oder ein Computerprogrammprodukt mit einem solchen Programm.

Die Erfindung wird nachfolgend anhand bevorzugter Ausführungsbeispiele beschrieben werden. Es zeigen:
- Figuren 1A und 1B: eine erste Ausführungsform einer erfindungsgemäßen Vorrichtung;
- Figur 2: die an Beckenknochen angelegte Vorrichtung gemäß Figur 1;
- Figur 3: eine zweite Ausführungsform einer erfindungsgemäßen Vorrichtung;
- Figur 4: eine Seitenansicht einer auf ein Becken aufgesetzten Vorrichtung gemäß der ersten Ausführungsform der Erfindung;
- Figuren 5 und 6: die Vorrichtung gemäß einer zweiten Ausführungsform während eines Einstellvorganges;
- Figur 7: eine Draufsicht auf die Vorrichtung gemäß der ersten Ausführungsform während eines Einstellvorganges;
- Figur 8: ein erfindungsgemäßes System; und
- Figur 9: die schematische Darstellung der Beckenknochen mit eingezeichneter Midsagittalebene und Beckeneingangsebene.

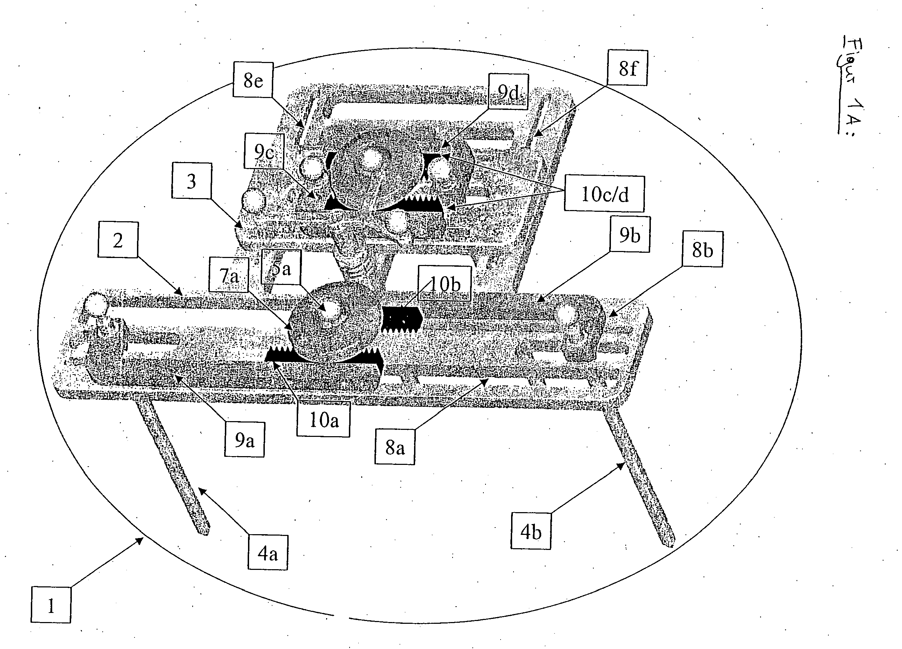

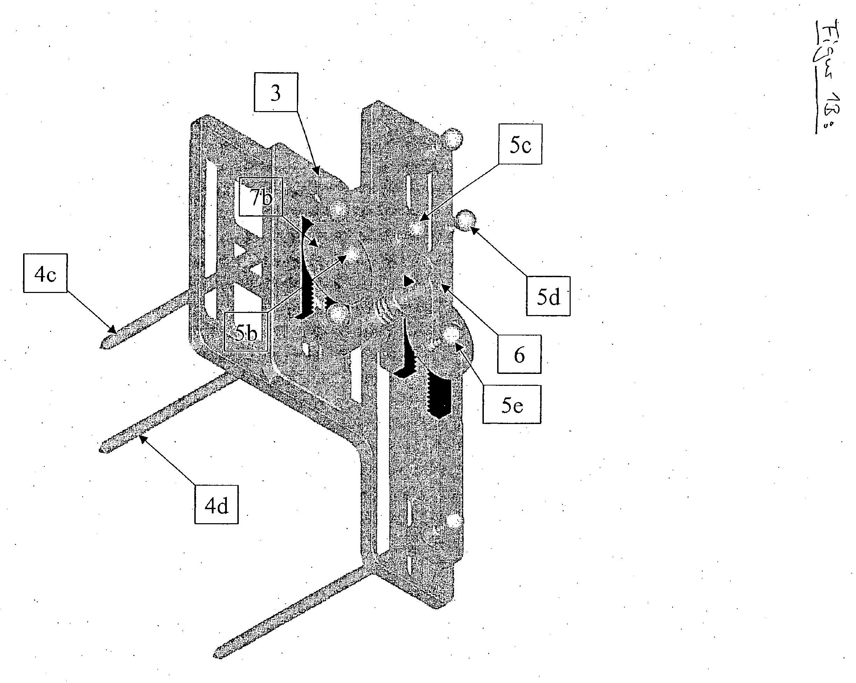

Figuren 1A und 1B zeigen eine Hüftregistrierungsvorrichtung 1, welche an einen Patienten in Seitenlage oder auch in Rückenlage angelegt werden kann, mit einer Platte 2, in welcher als Schlitze ausgebildete Führungen 8a und 8b zur Führung zweier Positionierungselemente 4a und 4b vorhanden sind. Die Positionierungselemente 4a und 4b werden von den Halterungen 9a und 9b gehalten, welche einander gegenüberliegend jeweils eine Zahnstange 10a und 1Ob aufweisen, die in ein unter einem Verstellelement 7a liegendes (nicht gezeigtes) Zahnrad eingreifen, so dass eine Drehung an dem Verstellelement 7a das gleichmäßige gegenläufige Bewegen beider Positionierungselemente 4a und 4b von dem Verstellelement 7a weg bzw. zu dem Verstellelement 7a hin bewirkt. Dabei liegt der Mittelpunkt des Verstellelements 7a bevorzugt immer in der Mitte zwischen den Positionierungselementen 4a und 4b. Auf dem Verstellelement 7a ist ein reflektierender kugelförmiger Marker 5a angebracht.

Die Platte 2 weist weiterhin Führungen 8e und 8f zur Führung eines Schlittens 3 auf, auf welchem ebenfalls über ein Verstellelement 7b verstellbar zwei mit Zahnstangen 10c und 10d versehene Halterungen 9c und 9d angeordnet sind, die mit Positionierungselementen 4c und 4d verbunden sind, wobei auch die auf dem Schlitten 3 gelagerten Positionierungselemente 4c und 4d durch eine Drehung des Verstellelementes 7b so verschoben werden können, dass ein auf dem Verstellelement 7b angeordneter Marker 5b immer in der Mitte zwischen den beiden Positionierungselementen 4c und 4d liegt.

Der Schlitten 3 kann entlang der Führungen 8e und 8f von den Positionierungselementen 4a und 4b weg oder auf diese zu bewegt werden, wobei die Positionierungselemente 4a bis 4d so gelagert oder geführt sind, dass die als Stäbe ausgebildeten Positionierungselemente zueinander in etwa immer parallel sind und wobei eine durch die als Anlagepunkte dienenden Unterseiten der Positionierungselemente 4a und 4b gebildete Linie immer parallel zu einer durch die als Anlagepunkte dienende Unterseite der Positionierungselemente 4c und 4d gebildete Linie ist.

Auf der Platte 2 ist ein Referenzstern 6 mit drei reflektierenden Markern 5c, 5d und 5e befestigt, wobei die Sternebene parallel zu einer durch die Unterseite der Positionierungselemente 4a bis 4d definierten Ebene ist.

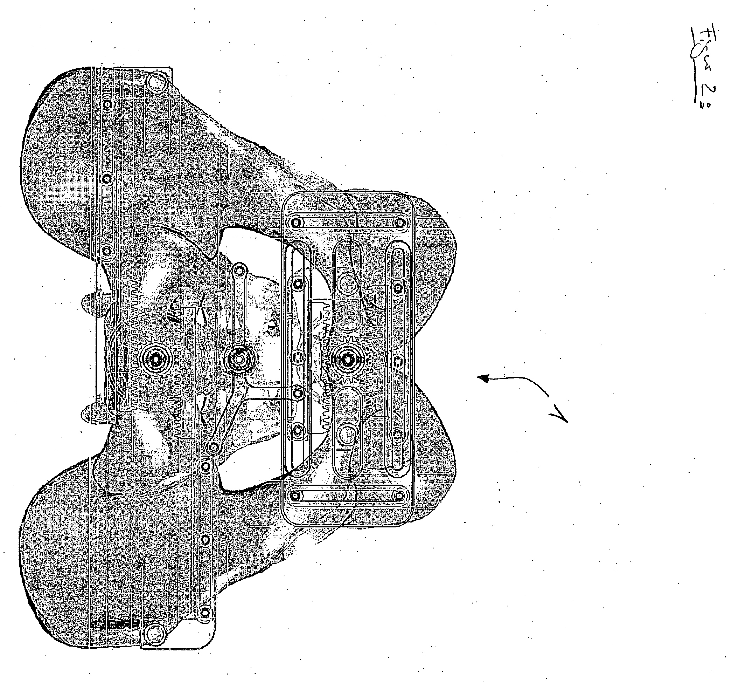

Somit können die Positionierungselemente 4a bis 4d der Registrierungsvorrichtung 1 so bewegt werden, dass diese auf die charakteristischen Punkte einer im wesentlichen symmetrisch ausgebildeten Hüfte aufgesetzt werden können, wie in Figur 2 gezeigt.

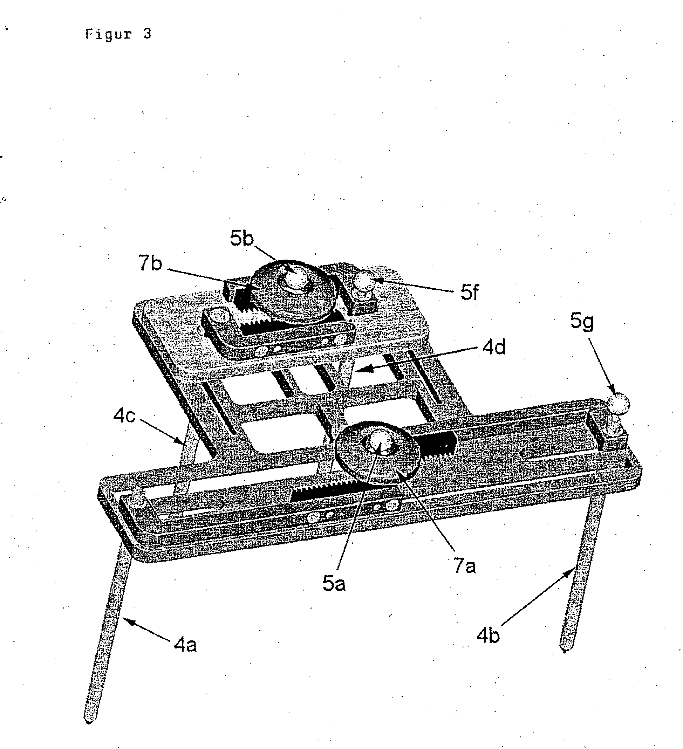

Figur 3 zeigt eine zweite Ausführungsform der erfindungsgemäßen Vorrichtung, wobei im Unterschied zur in Figur 1 und Figur 2 gezeigten ersten Ausführungsform kein Referenzstern 6 vorgesehen ist, sondern Marker 5f und 5g auf zwei Positionierungselementen 4b und 4d vorgesehen sind, welche auf einen Spinapunkt und einen Pubispunkt aufgesetzt werden.

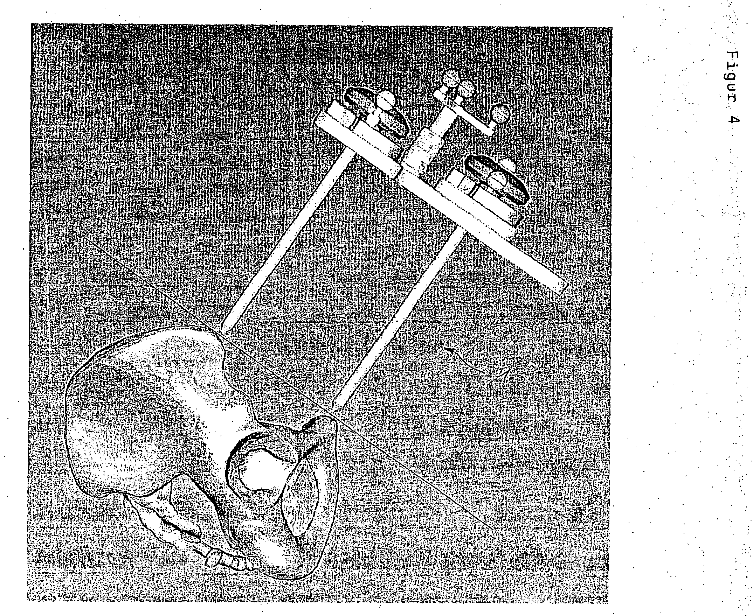

Sind die Positionierungselemente 4 auf charakteristische Punkte einer Hüfte aufgesetzt worden, wie in Figur 4 für eine Vorrichtung gemäß der ersten Ausführungsform gezeigt, so wird durch die Aufsetzpunkte der Positionierungselemente 4a bis 4d die Beckeneingangsebene bestimmt, welche einfach ermittelt werden kann, wenn die Ebene des Referenzsternes 6 parallel zu der durch die Anlagepunkte der Positionierungselemente 4 gebildeten Ebene liegt und die Geometrie der Registrierungsvorrichtung 1, d.h. der Abstand der Ebene des Referenzsternes 6 von der durch die Positionierungselemente 4 gebildeten Ebene, bekannt ist. Die Midsagittalebene kann aus der Position der auf den Verstellelementen 7a, 7b angeordneten Marker 5a, 5b und der Position eines weiteren Markers des Referenzsternes ermittelt werden. Somit kann die Lage der Midsagittal- und der Beckeneingangsebene bestimmt werden, wodurch die Lage der Beckenknochen bekannt und somit die Hüfte registriert ist.

Ebenso ist es möglich eine Hüfte mit der in Figur 3 gezeigten Vorrichtung zu registrieren, wobei ebenso wie bei der in Figur 1 und 2 gezeigten Vorrichtung die Positionierungselemente 4a bis 4d auf die Spina- und Pübispunkte aufgesetzt werden und die Position der Marker 5a, 5b, 5g und 5f ermittelt wird. Ist die Geometrie der Vorrichtung bekannt und liegen die Marker 5a, 5b, 5g und 5f zum Beispiel in einer Ebene, welche parallel zu der durch die Anlagepunkte der Positionierungselemente 4a bis 4d definierten Ebene ist, so kann die Lage der Beckeneingangsebene aus der erfassten Position der Marker 5a, 5b, 5f und 5d ermittelt werden. Ebenso kann die durch die Marker 5a und 5b verlaufende Midsagittalebene ermittelt werden, wenn die Position der Marker 5f und 5g berücksichtigt wird, insbesondere wenn die Marker 5a, 5b, 5f und 5b, 5a, 5g jeweils einen rechten Winkel definieren.

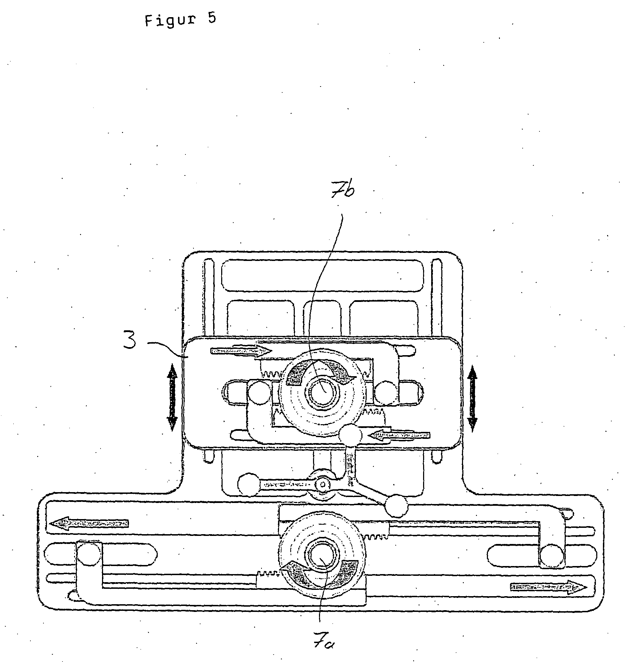

Figur 5 zeigt die in Figur 3 gezeigte Vorrichtung während eines Einstellvorgangs, wobei zunächst die als Positionierungselemente dienende Stäbe oder Pins 4a und 4b auf die Spinapunkte (ASIS) der Hüfte aufgesetzt werden. Der Abstand der Pins 4a und 4b voneinander kann durch eine Drehung des Verstellelements 7a eingestellt werden, wobei sich die beiden Pins 4a und 4b synchron so bewegen, dass das Einstellelement 7a und der darauf angeordnete Marker 5a immer in der Mitte zwischen den beiden Pins 4a und 4b liegt. Anschließend wird der Abstand zwischen den auf die Pubispunkte aufzusetzenden Pins 4c und 4d durch Drehung des Verstellelements 7b eingestellt. Falls es nicht möglich ist die Spitzen der Pins 4c und 4d exakt auf beide Pubispunkte aufzusetzen, kann nur der Pubispunkt der zu behandelnden Seite markiert werden, d.h. der Pin, welcher auf der nicht zu behandelnden Seite liegt, liegt nicht auf einem Pubispunkt auf. Anschließend wird der Abstand zwischen der durch die Pins 4a und 4b definierten "A-SIS-Linie" und der durch die Pins 4c und 4d definierten "Pubislinie" durch Verschieben des Schlittens 3 eingestellt.

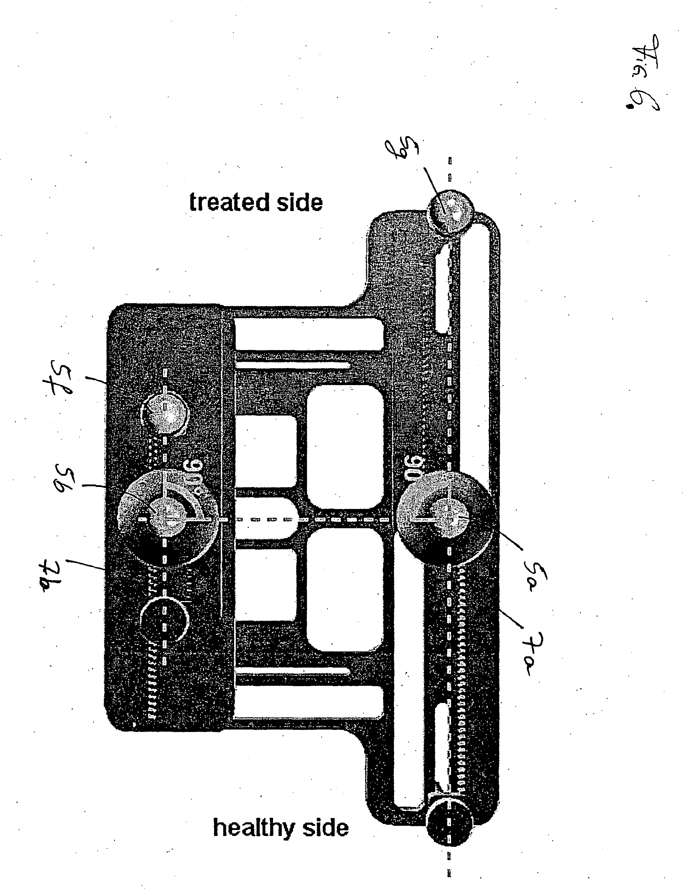

Figur 6 zeigt eine Ausführungsform mit vier Markern, wobei zwei Marker 5a, 5b auf den Verstellelementen 7a und 7b und zwei Marker 5f, 5g auf den Positionierungselementen 4a und 4c einer Seite angeordnet sind. Mit der gezeigten Vorrichtung können die Midsagittalebene, ein Spinapunkt und ein Pubispunkt auf der zu behandelnden Seite erfasst werden. Dabei muss ein Erfassungssystem oder eine Software vier einzelne Marker detektieren, welche vorteilhaft auf einer Ebene liegen, die parallel zu den Anlagepunkten der Positionierungselemente liegt. Die Midsagittalebene steht definitionsgemäß senkrecht auf dieser Ebene, wobei die Lage und Orientierung der Midsagittalebene zusätzlich durch die zwei in der Mitte der Vorrichtung angeordneten Marker bestimmt wird. Die vier Marker definieren zwei rechte Winkel. Die Position der Spina- und Pubispunkte auf der zu behandelnden Seite sind bekannt, wenn die Länge der verwendeten Positionierungselemente 4a und 4c, auf welchen die Marker 5f, 5g angeordnet sind, bekannt ist. Der Spina- und der Pubispunkt auf der nicht zu behandelnden gesunden Seite kann durch Spiegelung der durch die Positionierungselemente 4a und 4c erfassten Lage der Spina- und Pubispunkte an der Midsagittalebene ermittelt werden.

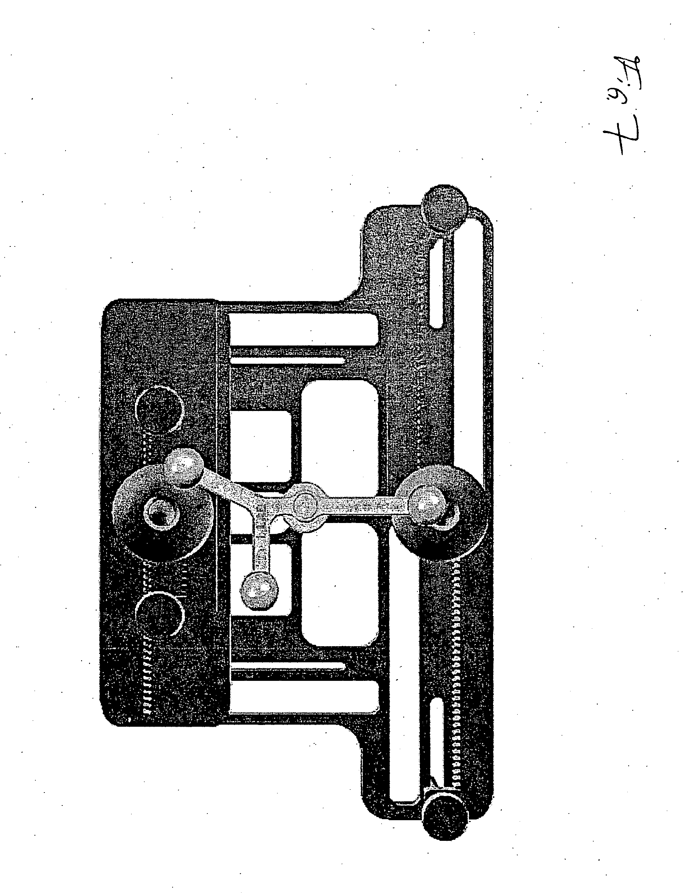

Figur 7 zeigt eine dritte Ausführungsform der erfindungsgemäßen Vorrichtung mit einem Referenzstern, wobei mit der gezeigten Ausführungsform nur zwei Hüftebenen detektiert werden können, so dass ein Benutzer mindestens zwei weitere Landsmarken erfassen muss, um die Hüfte zu registrieren.

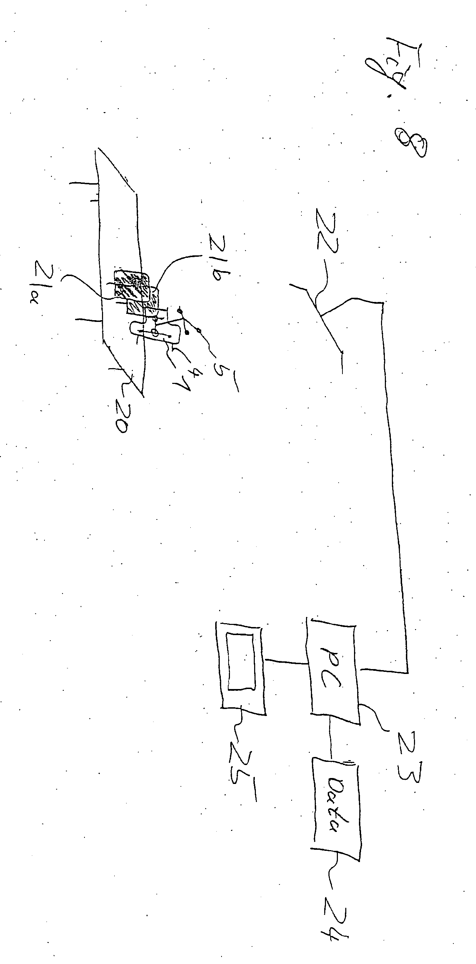

Figur 8 zeigt einen Tisch 20 mit darauf angeordneten gepolsterten Platten 21a und 21b, zwischen welchen ein Patient in Seitenlage gehalten werden kann. Ist ein Patient in Seitenlage durch die Platten 21a und 21b fixiert, kann mittels der an den Patienten angelegten Registrierungsvorrichtung 1 die Hüfte des Patienten registriert werden, wobei die Position der mit der Registrierungsvorrichtung 1 verbundenen Marker 5 durch eine Infrarotkamera 22 erfasst wird, welche die Position der Marker 5 an eine Recheneinheit 23 übermittelt, der aus einer Datenbank 24 Informationen zur Geometrie der Registrierungsvorrichtung 1 zugeführt werden, so dass daraus die Position der Spitzen der Positionierungselemente 4 der Registrierungsvorrichtung 1 berechnet werden kann, um aus den so gewonnen Informationen die Daten zur Hüftregistrierung zu gewinnen. An ei-, nem Bildschirm 25 kann zum Beispiel eine wie in Figur 9 gezeigte Information zur Registrierung der Hüfte ausgegeben werden, wobei die Lage der Midsagittalebene und der Beckeneingangsebene dargestellt werden kann.

## Patentansprüche

1. Vorrichtung zum Registrieren einer Hüfte eines Patienten mit Markern (5) und Positionierungselementen (4), welche auf charakteristische Punkte der Hüfte aufgesetzt werden können, wobei nach dem Aufsetzen der Positionierungselemente (4) auf charakteristische Punkte der Hüfte aus der Position der Marker (5) Daten zur Registrierung der Hüfte ermittelt werden können.

2. Vorrichtung nach Anspruch 1, wobei das mindestens eine Positionierungselement (4) ein Stab ist.

3. Vorrichtung nach einem der vorhergehenden Ansprüche, mit mindestens einem Verstellelement (7), um die Position mindesten eines Positionierungselementes (4) der Vorrichtung verstellen zu können.

4. Vorrichtung nach einem der vorhergehenden Ansprüche, wobei mindestens ein Verstellelement (7) so ausgebildet ist, dass die Position zweier Positionierungselemente (4) gleichzeitig und bevorzugt gegenläufig verstellt wird.

5. Vorrichtung nach einem der vorhergehenden Ansprüche, wobei die Marker (5) passive Marker und insbesondere reflektierende Oberflächen oder Kugeln sind.

6. Vorrichtung nach einem der vorhergehenden Ansprüche, wobei mit der Vorrichtung ein Referenzstern (6) verbunden ist, dessen Sternebene bevorzugt parallel zu einer Ebene ist, die durch die Anlagepunkte der Positionierungselemente (4) definiert ist.

7. Vorrichtung nach einem der vorhergehenden Ansprüche, wobei mindestens ein Marker (5) mit einem Positionierungselement (4) und/oder einem Verstellelement (7) verbunden ist und/oder mindestens zwei der durch drei Marker definierbaren Winkel 90 Grad betragen.

8. Vorrichtung nach einem der vorhergehenden Ansprüche, wobei mindestens ein Marker (5) über eine verstellbare Markeranordnung mit der Vorrichtung verbunden ist.

9. System mit einer Vorrichtung (1) nach einem der vorhergehenden Ansprüche, einer Erfassungseinheit (22) zur Erfassung der Position der Marker (5) der Vorrichtung (1), einer Recheneinheit (23), welche mit der Erfassungseinheit (22) verbunden ist und basierend auf Daten zur Geometrie der Vorrichtung (1) Informationen zur Registrierung einer Hüfte, an welcher die Vorrichtung (1) angelegt werden kann, ermitteln kann.

10. Verfahren zum Registrieren einer Hüfte mit einer Vorrichtung nach einem der Ansprüche 1 bis 8, wobei die Position der auf der Vorrichtung (1) angeordneten Marker (5) erfasst, daraus die Position der Anlagepunkte der Positionierungselemente (4) errechnet wird und daraus Daten zur Registrierung der Hüfte ermittelt werden.

11. Computerprogramm, welches, wenn es auf einem Computer läuft oder in einen Computer geladen ist, das Verfahren nach den vorhergehenden Anspruch durchführt.

12. Programmspeichermedium oder Computerprogrammprodukt mit dem Computerprogramm nach dem vorhergehenden Anspruch.
